# EUROPÄISCHE PATENTANMELDUNG

(11) **EP 1 493 822 A1**
(43) Veröffentlichungstag der Anmeldung: **05.01.2005**
(21) Anmeldenummer: 03015027.0
(22) Anmeldetag: 02.07.2003
(51) Int. Cl.: C12Q 1/68

(54) **Detektionsverfahren für Nucleinsäuren mit interner Amplifikationskontrolle**

(71) Anmelder: Labor Becker, Olgemoeller & Kollegen GbR, 81671 München (DE)
(72) Erfinder: Burggraf, Siegfried, 82194 Groebenzell (DE)
(74) Vertreter: Olgemöller, Luitgard, Dr.

(57) **Zusammenfassung**

Die vorliegende Erfindung betrifft ein Verfahren zum qualitativen oder quantitativen Nachweis einer Nucleinsäure in einer Probe mittels Amplifikation dieser Nucleinsäure und unter Verwendung einer oder mehrerer Detektions-Sonde(n), die reversibel an die amplifizierbare/amplifizierte Nucleinsäure binden kann/können und aufgrund dieser Eigenschaft eine Detektion der nachzuweisenden Nucleinsäure ermöglichen. Das Verfahren wird in Gegenwart einer ebenfalls zu amplifizierenden Kontroll-Nucleinsäure in dieser Probe durchgeführt, die dieselbe(n) Detektions-Sonde(n) wie die nachzuweisende Nucleinsäure bindet, aber im Bindungsbereich der Sonde(n) im Vergleich zur nachzuweisenden Nucleinsäure Abweichungen in der Nucleotid-Sequenz aufweist. Die Abweichungen sind derart, dass die Produkte aus nachzuweisender Nucleinsäure und Sonde(n) einerseits und Kontroll-Nucleinsäure und Sonde(n) andererseits einen unterschiedlichen Schmelzpunkt aufweisen, wobei die Temperaturdifferenz der Schmelzpunkte ausreichend groß ist, um die beiden Produkte analytisch voneinander unterscheiden zu können. Die Erfindung betrifft weiterhin für dieses Verfahren verwendbare Kontroll-Nucleinsäuren sowie Kits aus solchen Nucleinsäuren mit einer oder mehreren entsprechenden Sonde(n).

## Beschreibung

Die vorliegende Erfindung betrifft ein Verfahren zum qualitativen oder quantitativen Nachweis einer Nucleinsäure in einer Probe mittels Amplifikation dieser Nucleinsäure und unter Verwendung einer oder mehrerer reversibel bindender Detektions-Sonde(n), bei dem eine interne Nucleinsäure eingesetzt wird, die als Kontrolle falsch-negativer Ergebnisse dienen soll.

Der qualitative oder quantitative Nachweis von Nucleinsäuremolekülen, sei es DNA, sei es RNA, ist vor allem seit Auffinden PCR-geeigneter Polymerasen ein wichtiges Verfahren der labörmedizinischen Chemie. Hierfür werden die häufig in nur wenigen oder sogar nur einer Kopie vorliegenden target-Moleküle amplifiziert und mit Hilfe von Sonden detektiert, die das Vorhandensein dieser Moleküle anzeigen können.

Derartige Verfahren müssen gewissen Kontrollen unterliegen, die sicherstellen, dass sowohl das verwendete Amplifikationssystem die Amplifizierung und Detektion des nachzuweisenden Moleküls ermöglicht hätte, wäre es in der Probe vorhanden gewesen (Überprüfung falsch negativer Ergebnisse, d.h. Nachweis der Probenaufarbeitungseffizienz und Ausschluss einer PCR-Inhibition, z.B. durch Hemmung der Polymerase), als auch verifizieren, dass die verwendeten Chemikalien funktionsfähig waren (Positivkontrolle). Solche Kontrollen sind in vielen Bereichen insbesondere der Labormedizin inzwischen auch durch Normen vorgeschrieben (siehe auch die Qualitätsstandards in der mikrobiologisch-bakteriologischen Diagnostik MiQ 1/201: Nucleinsäureamplifikationstechniken).

Bei den Kontrollen ist zwischen externen (in einer getrennten Reaktionskammer ablaufenden) und internen (in der zu untersuchenden Probe durchgeführten) Kontrollen zu unterscheiden. Zur Feststellung, ob der Detektionstest richtig funktioniert (positive und negative Kontrolle), hat man früher vor allem externe Kontrollsysteme verwendet. Allerdings ist es insbesondere für den Nachweis, ob eine Inhibition der Amplifikation vorliegt, von Vorteil, eine interne Kontrolle zu verwenden. Interne Kontrollen können auch als Quantifizierungsstandard eingesetzt werden.

Sowohl für qualitative als auch quantitative Nachweise wird das interne Kontrollsystem vorzugsweise bereits zu einem sehr frühen Zeitpunkt zugesetzt, um möglichst alle im Zusammenhang mit dem Nachweis der gesuchten Nucleinsäure erfolgenden Schritte mit zu überprüfen.

Die interne Kontrolle erfolgt in der Regel durch Zugabe eines artifiziellen Nucleinsäure-Moleküls zu der Probe, in der die target-Nucleinsäure (das Zielmolekül) vermutet wird. Dieses artifizielle Molekül entspricht in der Basensequenz möglichst weitgehend dem zu amplifizierenden Nucleinsäure-Bereich und kann daher mit der gleichen Effizienz wie das eigentliche Zielmolekül amplifiziert werden. Detektiert wird es in der Regel unter Verwendung mindestens einer Oligonucleotid-Detektionssonde, die mit dem Zielmolekül hybridisieren kann und die eine Reportergruppe mit beobachtbaren Eigenschaften trägt, die sich in Abhängigkeit davon ändern, ob die Sonde am Zielmolekül gebunden oder von ihm abgelöst vorliegt. Zur Unterscheidung vom Zielmolekül ist derjenige Bereich des Kontroll-Nucleinsäure-Moleküls, der an die Detektionssonde(n) bindet, gegenüber dem entsprechenden Bereich des ersteren modifiziert; ein zu diesem modifizierten Bereich komplementäres Sondensystem wird eingesetzt, um das amplifizierte Kontroll-Nucleinsäure-Molekül zu detektieren. Dabei ist es notwendig, dass das zweite Sondensystem eine andere Reportergruppe enthält als das erste, z.B. einen bei einer anderen Wellenlänge absorbierenden Farbstoff, damit es durch eine Eigenschaft dieser Reportergruppe detektiert werden kann, die sich von der zu detektierenden Eigenschaft der ersten Reportergruppe unterscheidet.

Wünschenswert ist es, dass das Kontrollsystem dem zu detektierenden System möglichst ähnlich ist, um eine möglichst gute Vergleichbarkeit zu erhalten, insbesondere bei quantitativen Bestimmungen. Allerdings kann dies den Nachteil haben, dass infolge der hohen Homologie zwischen Kontroll-Nucleinsäure und nachzuweisender Nucleinsäure diese beiden sowie ihre Amplifikate miteinander kreuz-hybridisieren und damit falsche Ergebnisse liefern. In der EP 1 236 805 A1 ist ein Verfahren beschrieben, in dem eine Kontroll-Nucleinsäure eingesetzt wird, die zur nachzuweisenden Nucleinsäure parallelkomplementäre Strukturen besitzt. Die Eigenschaften dieser Kontrollen, darunter auch z.B. die Sekundärstrukturen, sind denen der nachzuweisenden Nucleinsäuren besonders ähnlich; die Kontrollen können aber nicht mit den zu detektierenden Molekülen hybridisieren, so dass derart falsche Ergebnisse vermieden werden. Allerdings müssen die Kontrollen mit eigens dafür geschneiderten Sonden detektiert werden. Im US-Patent 5,952,202 ist ein anderes Detektionssystem mit interner Kontrolle beschrieben, das auf der Detektion mit Reporter-Quencher-Sonden beruht, die während der Amplifikation von der verwendeten Polymerase verdaut werden. Dabei wird die Quenching-Gruppe von der Reportergruppe getrennt, und die Reportergruppe, beispielsweise eine fluoreszierende Gruppe, wird aktiviert. Ein internes Kontroll-Polynucleotid wird gleichzeitig mit internen Kontroll-Primern amplifiziert und mit Hilfe einer Reporter-Quencher-Sonde detektiert, die sich bezüglich ihrer spektrometrischen Eigenschaften von derjenigen des für den Nachweis der gesuchten Nucleinsäure eingesetzten Reporter-Quencher-Sonde unterscheidet. Bei diesem System muss das Kontroll-Nucleotid mit spezifisch hierfür geschneiderten Primern amplifiziert werden. Nachteilig ist an beiden Systemen zum einen, dass die Herstellung der zusätzlichen Komponenten (insbesondere der Sonden) Kosten verursacht. Zum zweiten zwingt die Detektion auf eine unterschiedliche physikalische Eigenschaft der Reportergruppe dieser Sonde(n) zum Einsatz eines zweiten Detektionssytems, z.B. eines eigenen Kanals zur Messung der Absorption/Fluoreszenz bei einer zweiten Wellenlänge.

Aufgabe der vorliegenden Erfindung ist es, eine vereinfachte interne Kontrolle für ein Nucleinsäure-Detektionsverfahren bereitzustellen, bei dem die Nucleinsäure amplifiziert wird.

Die Aufgabe wird dadurch gelöst, dass das Verfahren in Gegenwart einer ebenfalls amplifizierbaren Kontroll-Nucleinsäure durchgeführt wird, die die dieselbe(n) Detektions-Sonde(n) wie die nachzuweisende Nucleinsäure bindet, aber im Bindungsbereich der Sonde(n) im Vergleich zur nachzuweisenden Nucleinsäure Abweichungen in der Nucleotid-Sequenz aufweist, derart, dass das Produkt aus Kontroll-Nucleinsäure und Sonde(n) einen anderen Schmelzpunkt aufweist als das Produkt aus nachzuweisender Nucleinsäure und Sonde(n). Die Differenz der beiden Schmelzpunkte muss dabei so groß sein, dass sich die beiden Produkte durch eine Schmelzanalyse oder ein anderes Detektionsverfahren analytisch voneinander unterscheiden lassen. Günstig ist hierfür ein Wert von nicht weniger als ca. 5°C. Dieser letztere Wert macht es beispielsweise möglich, die Schmelztemperatur der Paarung aus nachzuweisender Nucleinsäure und Sonde(n) in einem Schmelzdiagramm oder dergleichen eindeutig von der Schmelztemperatur der Paarung aus Kontroll-Nucleinsäure und Sonde(n) zu unterscheiden. Eine eindeutige Detektion ermöglicht den Einsatz der Kontroll-Nucleinsäure als interne Kontrolle. Bei geringeren Temperaturdifferenzen als der angegebenen muss dagegen in einer Reihe von Fällen damit gerechnet werden, dass sich die Signale der gebundenen Sonden nicht mehr so deutlich unterscheiden, dass die Kontrolle als zuverlässig bewertet werden kann.

In einer bevorzugten Ausgestaltung der Erfindung liegt der Schmelzpunkt des Produktes aus Kontroll-Nucleinsäure und Sonde(n) niedriger, und gemäß den obigen Ausführungen besonders bevorzugt um mindestens 5°C niedriger als der des Produktes aus nachzuweisender Nucleinsäure und Sonde(n).

Erfindungsgemäß wird dasselbe Sondensystem (eine oder mehrere Sonden) für die Detektion der nachzuweisenden Nucleinsäure und der Kontroll-Nucleinsäure verwendet. Dies spart einerseits Kosten; andererseits muss/müssen die Sonde(n) für die Kontroll-Nucleinsäure nicht über ein zweites Detektionssystem nachgewiesen werden. Außerdem entfällt bei Einsatz der vorliegenden Erfindung die Notwendigkeit, eine Positivkontrolle zu verwenden, die das kontrollierende Sondensystem ihrerseits auf seine Effizienz kontrolliert. Denn wie voranstehend ausgeführt, benötigt die erfindungsgemäße interne Amplifikationskontrolle kein zweites Sondensystem; und damit entfällt auch dessen eigenständige Kontrolle.

Die vorliegende Erfindung ist für alle Sondensysteme geeignet, die während der Amplifikation nicht verbraucht, z.B. verdaut werden.

Dem Fachmann sind die Maßnahmen zur Erzielung einer geeigneten Schmelztemperatur zwischen Kontroll-Nucleinsäure und Sonde(n) bekannt; überschlägig gilt die folgende Bindungsregel für eine gut bindende Sonde (ca. 20 Basen): Die Nucleotidbausteine G und C (mit je drei Wasserstoff-Brückenbindungen) tragen mit je ca. 4°C, die Nucleotidbausteine A und T (mit je zwei Wasserstoff-Brückenbindungen) tragen mit je ca. 2°C zur Annealing-Temperatur bei, sofern sie an der Bindung beteiligt sind (Wallace/Ikutana Regel Tm (°C) = 2x(#A+#T)+4x(#G+#C), zitiert in Wetmur J.G., 1991, Crit. Rev. Biochem. Mol. Biol. 26:227-259)). Genauere Vorhersagen sind mit der sogenannten "Nearest Neighbor" Methode möglich, die auf thermodynamischen Berechnungen beruht (Borer P.N. et. al., 1974, J. Mol. Biol. 86:843-853, modifiziert durch Allawi, H.T. et al., 1997, Biochemistry 36:10581-594). Die Einführung einer Fehlbindungsstelle ("mismatch") senkt den Schmelzpunkt meist grob um 5 bis 9°C. Dabei hat die Art des mismatches einen starken Einfluss auf die Schmelzpunktsänderung. So verursacht ein GT-mismatch nur eine sehr geringe Reduktion des Schmelzpunktes, ein CA-mismatch die stärkste. Die Wirkung der übrigen Unpaarungen liegt zwischen diesen beiden, und zwar in der folgenden Reihenfolge: GT<GA<TT<GG<AA<CC<CT<CA. Zusätzlich ist die Senkung stark von der Position des mismatches im Bindungsbereich und von der Basenzusammensetzung in der Umgebung des mismatches beeinflusst. Die Anwendung der "Nearest Neighbor" Methode auf in sog. LightCycler Verfahren (Real time Verfahren der Fa. Roche) verwendbare Sonden und der Einfluss von mismatches ist ausführlich beschrieben in Von Ahsen, N., Schütz E. (2001), Using the nearest neighbor model for the estimation of matched and mismatched hybridization probe melting points and selection of optimal probes on the LightCycler. In: Rapid cycle real-time PCR, methods and applications, Meuer, S., Wittwer, C., Nakagawara, K. (eds), pp. 433-56, Springer Verlag Berlin, Heidelberg, New York.

Die vorstehenden Erläuterungen zeigen, dass es in vielen Fällen ausreichend sein wird, in der Nucleotid-Sequenz der Kontroll-Nucleinsäure innerhalb des Bindungsbereichs der Detektions-Sonde(n) nur ein Nucleotid, das in der nachzuweisenden Nucleinsäure vorhanden ist, gegen ein anderes auszutauschen. Vorzugsweise finden sich darin aber mindestens zwei Abweichungen. Um die Sondenbindung an die Kontroll-Nucleinsäure im übrigen möglichst vergleichbar zur Bindung der Sonde an die nachzuweisende Nucleinsäure zu machen, ist es dabei günstig, wenn die "mismatches" möglichst gleichmäßig über den Bindungsbereich verteilt angeordnet werden.

Das Verfahren ist für die Detektion verschiedener Arten von Nucleinsäuren wie z.B. DNA oder RNA gleichermaßen einsetzbar. So kann es z.B. für die Detektion von erregerspezifischer Nucleinsäure verwendet werden, beispielsweise für den Nachweis von Viren, Bakterien, Pilzen u.ä. Konkrete Beispiele sind der Nachweis von verschiedenen Hepatitis-Viren, Herpes, Papilloma-Viren, Bordetellen, HIV, Coronaviren (Auslöser von SARS).

Die Kontroll-Nucleinsäure wird in vorteilhafter Weise der zu untersuchenden Probe möglichst frühzeitig zugesetzt. Günstig ist es beispielsweise, sie bereits der frisch dem Patienten entnommenen Probe (Blut, Urin oder dgl.) beizugeben.

Die Kontroll-Nucleinsäure ist in bevorzugter Weise eine einzelsträngige Nucleinsäure, z.B. ein Oligonucleotid, das in Gegenwart der für die Amplifizierung der nachzuweisenden Nucleinsäure erforderlichen Substanzen ebenso amplifiziert wird wie diese. Beispielsweise und bevorzugt kann die Erfindung auf Nachweise angewendet werden, die sich der gängigen PCR-Amplifikationstechnik bedienen. In diesen Fällen ist es günstig, wenn die Kontroll-Nucleinsäure eine solche Nucleotid-Abfolge am 3' und am 5'-Ende aufweist, dass die Amplifizierung des Nucleinsäurestranges genauso wie die des Gegenstranges in Gegenwart derselben Primer abläuft, die für die Amplifizierung der nachzuweisenden Nucleinsäure verwendet werden. Es sollte jedoch klar sein, dass diese bevorzugte Ausgestaltung günstig ist, weil ein ansonsten hinzutretender Aufwand für die Entwicklung der Nachweis-Bedingungen einschließlich dieser zusätzlichen Komponenten unterbleibt und weil die Komplexizität der Probe dadurch nicht erhöht wird (zusätzliche Komponenten erhöhen u.a. die Wahrscheinlichkeit einer ungewollten Primer/Primer-Paarung). Für die Erfindung ist diese Ausgestaltung jedoch kein notwendiges Kriterium.

Die Zahl und Auswahl der Basen für diejenigen Bereiche der Kontroll-Nucleinsäure, die weder für die Primer-Bindung noch für die Sondenbindung benötigt werden, ist von untergeordneter Bedeutung. Dies ist besonders überraschend, weil im Stand der Technik immer wieder betont wird, dass Kontroll-Nucleinsäuren den nachzuweisenden Nucleinsäuren so ähnlich wie möglich sein sollten. Und es ist vorteilhaft, weil die Herstellung kürzerer Kontroll-Nucleinsäuren wesentlich preiswerter ist.

In der Regel ist es nicht erforderlich, dass die Kontroll-Nucleinsäure in identischer Kopienzahl amplifiziert wird, solange einerseits eine Amplifizierung sichergestellt ist, die die zuverlässige Detektion der Kontroll-Nucleinsäure in der Testflüssigkeit (Probe) erlaubt, und andererseits ihre Amplifizierung gegenüber der Amplifizierung der nachzuweisenden Nucleinsäure nicht derart bevorzugt wird, dass die letztere ein signifikant verschlechtertes Ergebnis liefert. Es hat sich erfindungsgemäß herausgestellt, dass die genannten Bereiche der Kontroll-Nucleinsäure häufig stark abweichen und in einer Reihe von Fällen sogar signifikant kürzer, d.h. auch großenteils deletiert sein können, ohne dass die eine oder die andere der oben genannten Randbedingungen überschritten worden wäre. Diese Bedingungen können gegebenenfalls auch über die Steuerung des Verhältnisses der eingesetzten Kopienzahl von nachzuweisender Nucleinsäure und Kontroll-Nucleinsäure eingehalten werden.

Erfindungsgemäß eignet sich die Verwendung der Kontroll-Nucleinsäure sowohl für quantitative als auch für qualitative Nachweise von Nucleinsäure(n). Für einen qualitativen Nachweis können die Amplifikation der nachzuweisenden Nucleinsäure und die der Kontroll-Nucleinsäure, gegebenenfalls mit denselben Primern, in der gewünschten Zyklenzahl durchgeführt werden. Die Detektion der nachzuweisenden Nucleinsäure und die der internen Kontrolle erfolgen in einem Schmelzschritt nach der Amplifikation. Das einsträngige bzw. einsträngig gemachte (denaturierte) Amplifikat wird auf eine Temperatur abgekühlt, bei der die Sonde(n) an beide Nucleinsäuren bindet/binden. Danach erfolgt eine Temperaturerhöhung unter Detektion der beobachtbaren Eigenschaften des Sondensystems, die sich durch die Ablösung der Sonde(n) vom Nucleinsäure-Strang verändern. Das mit der/den Sonde(n) hybridisierte Amplifikat der internen Kontrolle zeigt aufgrund der Fehlpaarungen einen anderen, vorzugsweise niedrigeren Schmelzpunkt als das mit der/den Sonde(n) hybridisierte Amplifikat der nachzuweisenden Nucleinsäure.

Bei der qualitativen wie auch der quantitativen Bestimmung kann die Amplifikation der Kontroll-Nucleinsäure "still" im Hintergrund laufen; es ist deshalb auch nicht entscheidend, dass sie mit derselben Effizienz abläuft wie bei der nachzuweisenden Nucleinsäure.

Das Vorhandensein einer nachzuweisenden Nucleinsäure in quantitativen Verfahren wird in der Regel bei einer Temperatur detektiert, die etwas unterhalb ihrer Schmelztemperatur mit der oder den vorhandenen Sonde(n) liegt. Der Schmelzpunkt des Produktes aus Kontroll-Nucleinsäure und Sonde(n) sollte für quantitative Bestimmungen möglichst so gewählt werden, dass die beiden Komponenten bei dieser Temperatur im wesentlichen nicht hybridisieren. Aufgrund dieser Maßnahme stört das Kontrollsystem die Beobachtung der nachzuweisenden Nucleinsäure nicht. Gegebenenfalls kann die Detektionstemperatur im Verfahren relativ nahe an der Schmelztemperatur des Produkts aus nachzuweisender Nucleinsäure und Sonde(n) liegend gewählt werden, damit der Abstand zur Schmelztemperatur von Sonde(n) und Kontroll-Nucleinsäure ausreichend ist.

Dies gilt insbesondere für die sogenannten "Real time" oder Echtzeit-Verfahren, bei denen das Anwachsen der nachzuweisenden Nucleinsäure während der PCR beobachtet werden kann, beispielsweise mit dem sog. "LightCycler"-System der Firma Roche. Bei diesem besteht das Sondensystem aus zwei Sonden, von denen eine einen fluoreszierenden Farbstoff, die andere einen im sichtbaren Spektrum emittierenden Farbstoff aufweist. Einer der Farbstoffe befindet sich am oder in der Nähe des 3'-Endes der ersten Sonde, der andere am oder in der Nähe des 5'-Endes der zweiten Sonde, wobei die Sonden jeweils so geschneidert sind, dass sie im an die Nucleinsäure gebundenen Zustand nebeneinander zu liegen kommen, und zwar so, dass sich die Farbstoffe in räumlicher Nähe zueinander befinden. Dies führt zu einer Übertragung der Fluoreszenz-Resonanzenergie vom Fluoreszenz-Farbstoff auf den lichtabsorbierenden Farbstoff (die Reportergruppe), der dadurch zum Leuchten angeregt wird. Die Wellenlänge dieser Emission wird detektiert. Die Annealing- und die Schmelztemperatur von nachzuweisender Nucleinsäure und Primern bzw. Sondensystem liegen beide unterhalb der Arbeitstemperatur der Polymerase. Die gesuchte Nucleinsäure wird deshalb in Arbeitszyklen amplifiziert, die die Denaturierung der doppelsträngigen Nucleinsäure bei einer entsprechend hohen Temperatur, die Abkühlung auf die Annealing-Temperatur, bei der die Primer das Sondensystem binden und die Nucleinsäure detektiert wird, und eine Anhebung der Temperatur auf die Arbeitstemperatur der Polymerase umfasst, bei der ein neuer Gegenstrang an die Nucleinsäure ansynthetisiert wird.

Dieses Detektionsprinzip wurde bisher üblicherweise in Gegenwart eines Kontrollsystems mit einer synthetischen Nucleinsäure, für deren Amplifizierung erforderlichen Primern und einem aus zwei Sonden bestehenden, komplementären Sondensystem durchgeführt, das einen bei einer anderen Wellenlänge emittierenden Farbstoff aufweist und zusätzlich bei dieser zweiten Wellenlänge detektiert wird. Die vorliegende Erfindung lässt sich auf dieses Prinzip anwenden, indem statt dessen eine Kontroll-Nucleinsäure geschneidert wird, die dieselben Sonden bindet wie die nachzuweisende Nucleinsäure, in der aber eine oder einige Basen derart ausgetauscht sind, dass die Schmelztemperatur der Paarung aus Kontroll-Nucleinsäure und Sonden den erfindungsgemäßen, oben beschriebenen Bedingungen genügt. Im übrigen bindet die Sonde aber sterisch unverändert an die Kontroll-Nucleinsäure, so dass ihre Reportergruppe(n) in Kombination mit der Kontroll-Nucleinsäure dasselbe Signal ergeben, wie es von der Kombination der Sonde mit der nachzuweisenden Nucleinsäure abgegeben wird.

Das voranstehend beschriebene Prinzip lässt sich in entsprechend abgewandelter Form natürlich auch auf alternative Sondensysteme anwenden. Ein Beispiel hierfür ist das System der "molcular beacons". Dieses System arbeitet mit nur einer Sonde, die einen Farbstoff, z.B. einen Fluoreszenzfarbstoff, sowie eine Quenchergruppe enthält. In freiem Zustand faltet sich die Sonde so auf, dass die Quenchergruppe der Farbstoffgruppe nahe ist und deren Emission unterdrückt. In an die Nucleinsäure gebundenem Zustand befinden sich die Farbstoff- und die Quenchergruppe an räumlich auseinanderliegenden Teilen der Sonde, die dementsprechend fluoresziert. Auch bei diesem Detektionssystem kann eine interne Kontroll-Nucleinsäure eingesetzt werden, die die Sonde gleichermaßen bindet wie die nachzuweisende Nucleinsäure, deren Paarung mit der Sonde allerdings aufgrund von "mismatches" wie oben beschrieben einen niedrigeren Schmelzpunkt aufweist. Die Erfindung ist aber auch mit allen anderen bekannten oder noch zu entwickelnden Sondensystemen verwendbar, die während der Amplifikation nicht verbraucht werden.

Die vorliegende Erfindung kann sehr gut auch für sogenannte Multiplex-Analysen verwendet werden, bei denen mehrere Nucleinsäuren nebeneinander detektiert werden, z.B. bei der Suche nach bestimmten Erregervarianten. Da das erfindungsgemäße Kontrollsystem kein zweites Detektionssytem benötigt, können beispielsweise zwei Nucleinsäuren mit Hilfe von zwei Primer-Paaren und zwei Sondensystemen detektiert werden, wobei die beiden Sondensystemen über zwei verschiedene Reportergruppen detektiert werden, z.B. durch Beobachtung zweier unterschiedlicher Wellenlängen, bei denen diese Reportergruppen Licht emittieren. Erfindungsgemäß werden einem solchen System zwei interne Kontroll-Nucleinsäuren zugesetzt, von denen jede in Hinblick auf eine der nachzuweisenden Nucleinsäuren wie oben beschrieben geschneidert ist. Eine derartige Duplex-Analyse kann mit gängigen Zwei-Kanal-Meßapparaturen durchgeführt werden, da nicht mehr als die beiden Farbstoffe der Sondensysteme für die nachzuweisenden Nucleinsäuren detektiert werden müssen. Entsprechend sind mit Mehrkanal-Vorrichtungen höhere Multiplex-Analysen möglich.

Nachstehend soll die Erfindung anhand eines **Beispiels** näher erläutert werden, das die Verwendung der erfindungsgemäßen Kontrolle anhand des Nachweises von Hepatitis B-Virus zeigt.

Hepatitis B DNA wird durch Amplifikation eines 188 Basenpaare großen Abschnittes aus dem GenX nachgewiesen. Der Abschnitt besteht aus der folgenden Basenabfolge (SEQ ID NO:1):

5'GACGTCCTTTGTTTACGTCCCGTCGGCGCTGAATCCTGCCGACGACCCTTCTCGGGGCCGCT
TGGGGCTCTACGCCCTCTTTGCCGTCTGCCGTTCCAGCCAACCACGGGGCGCACCTCTCTTTAC
GCGGTCTCCCCGTCTGTGCCTTCTCATCTGCCGGTCCGTGTGCACTTCGCTTCACCTCTGCA3'

Diese Sequenz gliedert sich die folgenden Bereiche:

5'GACGTCCTTTGTTTACGTCCCGTC 3' (1)

5'GGCGCTGAATCCTGCCGAC 3' (2)

5'GACCCTTCTCGGGGCCGCTTGGGGCTCTACGCCCTCTTTGCCGTCTGCCGTTCCAGCCAACC
3' (2a)

5'ACGGGGCGCACCTCTCTTTACGCGG 3' (3)

5'T 3' (4)

5'CTCCCCGTCTGTGCCTTCTCATCTGC 3' (5)

5'C 3'(6)

5'GGTCCG 3' (6a)

5'TGTGCACTTCGCTTCACCTCTGCA 3' (7)

Bei 1 und 7 handelt es sich um die Primerbindungsbereiche. Mit 3 ist der Bereich beziffert, der mit der 3'-Fluoreszein-markierten Sonde hybridisiert; der Bereich, der mit der 5' Red640-markierten Sonde hybridisiert, trägt die Ziffer 5. Die restlichen Ziffern geben den übrigen Sequenzbereich wieder.

Der Test wird nach Jursch et al., Med. Microbiol. Immunol (Berl), 2002 Mar, 190(4):189-197, durchgeführt:

F-Primer:    5′ GAC GTC CTT TGT YTA CGT CCC GTC 3′

R-Primer: 5′ TGC AGA GGT GAA GCG AAG TGC ACA 3′

Ankersonde: 5′ ACG GGG CGC ACC TCT CTT TAC GCG G Fluorescein 3′

Detektionssonde: 5′ LC-Red640 CTC CCC GTC TGT GCC TTC TCA TCT GC
Phosphat 3′

Die Nukleinsäurepräparation erfolgt über Silicamembransäulen (Firma Qiagen) aus 200 µl Plasma oder Serum. Elution der DNA von der Säule in 50 µl.
PCR-Ansatze in Glaskapillaren der Firma Roche (Gesamtvolumen 10 µl) :
5 pmol von jedem Primer und 2 pmol von jeder Sonde
1 µl LightCycler-FastStart DNA Master Hybridization Probes (enthält Polymerase und Nukleotide)
Proben-DNA: 2,5 µl

Laufbedingungen im LightCycler (Roche):
1. DNA-Denaturierung und Aktivierung der Polymerase:
   Cycles "1"; Analysis Mode "None"
   Segment 1: Target Temperature "95"; Incubation Time "10:00";
   Temperature Transition Rate "20.0"; Secondary Target
   Temperature "0"; Step Size "0.0"; Step Delay "0"; Aquisition Mode "NONE"
2. Präcycling-Programm:
   Cycles "10"; Analysis Mode "None"
   Segment 1: Target Temperature "95"; Incubation Time "10;
   Temperature Transition Rate "20.0"; Secondary Target
   Temperature "0"; Step Size "0.0"; Step Delay "0"; Aquisition Mode "NONE"
   Segment 2: Target Temperature "64"; Incubation Time "8";
   Temperature Transition Rate "20.0"; Secondary Target
   Temperature "54"; Step Size "1.0"; Step Delay "0";
   Aquisition Mode "NONE"
   Segment 3: Target Temperature "72"; Incubation Time "13";
   Temperature Transition Rate "20.0"; Secondary Target
   Temperature "0"; Step Size "0.0"; Step Delay "0"; Aquisition Mode "NONE"
3. Hauptcycling-Programm:
   Cycles "35"; Analysis Mode "Quantification"
   Segment 1: Target Temperature "95"; Incubation Time "10";
   Temperature Transition Rate "20.0"; Secondary Target
   Temperature "0"; Step Size "0.0"; Step Delay "0"; Aquisition Mode "NONE"
   Segment 2: Target Temperature "54"; Incubation Time "8";
   Temperature Transition Rate "20.0"; Secondary Target
   Temperature "0"; Step Size "0.0"; Step Delay "0"; Aquisition Mode " NONE "
   Segment 3: Target Temperature "72"; Incubation Time "13";
   Temperature Transition Rate "20.0"; Secondary Target
   Temperature "0"; Step Size "0.0"; Step Delay "0"; Aquisition Mode " SINGLE "

Im Hauptcycling-Programm binden beim sogenannten
Annealingschritt (Segment 2: "TargetTemperature" 54°C) die beiden Primer und intern die beiden Sonden. Der grüne Fluoreszenzfarbstoff der Ankersonde (Fluorescein) wird angeregt und überträgt die Energie auf den roten Farbstoff (LightCycler Red 640) der Detektionssonde (**F**luoreszenz**R**esonanz**E**nergie**T**ransfer-Technolgie). Die von der Detektionssonde emittierte rote Fluoreszenz wird bei jedem Annealingschritt gemessen und korrespondiert mit der Zunahme an gebildetem PCR-Produkt.

### Interne Kontrolle:

Zur Überprüfung der Effizienz der Nukleinsäurepräparation und zum Ausschluss von falsch negativen Resultaten durch Hemmung der PCR-Reaktion wird folgendes Oligonukleotid mit 120 Basen als interne Kontrolle eingesetzt (SEQ ID NO:2)

5′GACGTCCTTTGTTTACGTCCCGTCGGCGCTGAATCCTGCCGACACGGGGCGCACCTCTCTTT
ACGCGGTCTACCAGTCTATGCCTTATCATCTGCCTGTGCACTTCGCTTCACCTCTGCA 3′

Diese Sequenz gliedert sich die folgenden Bereiche:

5'GACGTCCTTTGTTTACGTCCCGTC 3' (1")

5'GGCGCTGAATCCTGCCGAC 3' (2")

5'ACGGGGCGCACCTCTCTTTACGCGG 3' (3")

5'T 3' (4")

5'CTACCAGTCTATGCCTTATCATCTGC 3' (5")

5'C 3' (6")

5'TGTGCACTTCGCTTCACCTCTGCA 3' (7")

Die Primerbindungsstellen sind mit 1" und 7" bezeichnet; sie sind mit denen der Hepatitis-DNA identisch. Der mit 3" bezeichnete Bereich, der mit der 3'-Fluoreszein-markierten Sonde hybridisiert, ist ebenfalls identisch. Der Bereich, der mit der 5'Red640-markierten Sonde hybridisiert (5"), zeigt insgesamt vier Abweichungen gegenüber der nativen Hepatitis-DNA (viermal A statt C; G; G; C); diese Abweichungen betreffen das dritte, 6. 11. und 18. Nucleotid dieses Bereichs. Die Bereiche 2", 4" und 6" geben den übrigen Sequenzbereich wieder. Er korrespondiert mit den Bereichen 2, 4 und 6 in der oben gezeigten nativen Hepatitis-DNA; der dort mit 2a und 6a bezeichnete Bereich ist in der Kontroll-DNA deletiert.

Es werden 2,5 µl des Oligonukleotids (Konzentration 1000 Kopien/µl) zu 200 µl Plasma oder Serum zugegeben. Danach wird die Nukleinsäurepräparation wie oben beschrieben durchgeführt. Die PCR-Ansätze werden wie oben beschrieben durchgeführt. Es ist lediglich eine geringe Modifikation der LightCycler-Laufbedingungen notwendig:
1. DNA-Denaturierung und Aktivierung der Polymerase:
   Cycles "1"; Analysis Mode "None"
   Segment 1: Target Temperature "95"; Incubation Time "10:00";
   Temperature Transition Rate "20.0"; Secondary Target
   Temperature "0"; Step Size "0.0"; Step Delay "0"; Aquisition Mode "NONE"
2. Präcycling-Programm:
   Cycles "10"; Analysis Mode "None"
   Segment 1: Target Temperature "95"; Incubation Time "10"; Temperature Transition Rate "20.0"; Secondary Target
   Temperature "0"; Step Size "0.0"; Step Delay "0"; Aquisition Mode "NONE"
   Segment 2: Target Temperature "64"; Incubation Time "8";
   Temperature Transition Rate "20.0"; Secondary Target
   Temperature "54"; Step Size "1.0"; Step Delay "0"; Aquisition Mode "NONE"
   Segment 3: Target Temperature "72"; Incubation Time "13";
   Temperature Transition Rate "20.0"; Secondary Target
   Temperature "0"; Step Size "0.0"; Step Delay "0"; Aquisition Mode "NONE"
3.. Hauptcycling-Programm:
   Cycles "35"; Analysis Mode "Quantification"
   Segment 1: Target Temperature "95"; Incubation Time "10";
   Temperature Transition Rate "20.0"; Secondary Target
   Temperature "0"; Step Size "0.0"; Step Delay "0"; Aquisition Mode "NONE"
   Segment 2: Target Temperature "54"; Incubation Time "8";
   Temperature Transition Rate "20.0"; Secondary Target
   Temperature "0"; Step Size "0.0"; Step Delay "0"; Aquisition Mode "NONE "
   Segment 3: Target Temperature "60"; Incubation Time "0";
   Temperature Transition Rate "20.0"; Secondary Target
   Temperature "0"; Step Size "0.0"; Step Delay "0"; Aquisition Mode " SINGLE"
   Segment 4: Target Temperature "72"; Incubation Time "13";
   Temperature Transition Rate "20.0"; Secondary Target
   Temperature "0"; Step Size "0.0"; Step Delay "0"; Aquisition Mode "NONE"
4. Schmelzprogramm:
   Cycles "1"; Analysis Mode "Melting Curves"
   Segment 1: Target Temperature "95"; Incubation Time "5";
   Temperature Transition Rate "20.0"; Secondary Target
   Temperature "0"; Step Size "0.0"; Step Delay "0"; Aquisition Mode "NONE"
   Segment 2: Target Temperature "40"; Incubation Time "20";
   Temperature Transition Rate "20.0"; Secondary Target
   Temperature "0"; Step Size "0.0"; Step Delay "0"; Aquisition Mode "NONE "
   Segment 3: Target Temperature "85"; Incubation Time "0";
   Temperature Transition Rate "0.20"; Secondary Target
   Temperature "0"; Step Size ""0.0"; Step Delay "0"; Aquisition Mode "CONT"

Modifikation: Anhebung der Fluoreszenzmesstemperatur auf 60°C Zusätzliches Schmelzprogramm nach Abschluss der PCR-Amplifikation.

Die interne Kontrolle wird genauso wie Hepatitis B DNA bei der PCR amplifiziert, wird jedoch während der PCR nicht detektiert, da bei der Messtemperatur von 60°C keine Bindung der Detektionssonde an die interne Kontrolle erfolgt.

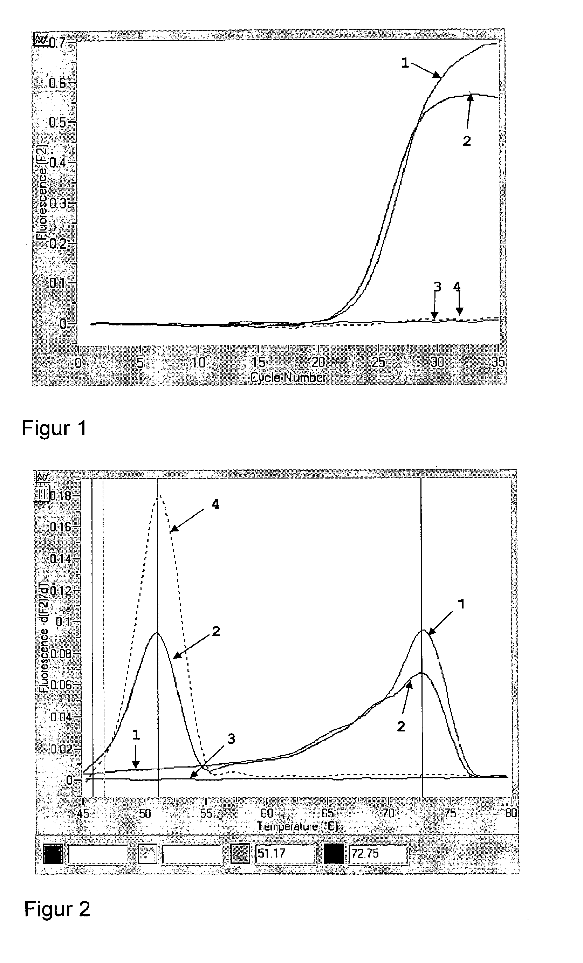

**In Figur 1** ist die Detektion des erregerspezifischen Amplifikats beim Annealingschritt der PCR zu sehen. Bei 60°C binden beide Detektionssonden an die erregerspezifische Zielsequenz. Es kommt zur Übertragung von Fluoreszenzresonanzenergie von Fluorescein auf Red640. Das rote Signal wird detektiert. Die Figur zeigt die Zunahme des Fluoreszenzsignals im Laufe der PCR-Amplifikation. Linie 1 zeigt die Amplifikation von 10.000 Kopien HBV-DNA ohne interne Kontrolle; Linie 2 zeigt die Amplifikation dieser Kopien mit Kontrolle. Die Linien 3 und 4 sind Negativkontrollen (ohne DNA) mit (Linie 4) und ohne (Linie 3) interner Kontrolle. Wie die Linie 4 zeigt, ergeben die Sonden mit der internen Kontrolle bei den gewählten Bedingungen (Messtemperatur 60°C) kein Signal, da eine Bindung der Red640-markierten Sonde aufgrund der Fehlpaarungen nicht möglich ist. Die Figur.zeigt, dass die Zugabe der internen Kontrolle keinen Einfluss auf das Resultat der PCR hat. Die Ansätze mit und ohne interne Kontrolle zeigen identische Ergebnisse. Die interne Kontrolle wird während der PCR-Amplifikation nicht detektiert.

Die interne Kontrolle lässt sich durch eine Schmelzanalyse nach Abschluss der PCR detektieren. Hierbei wird das Amplifikat zunächst bei 95°C denaturiert. Durch die abschließende Abkühlung auf 40°C ("Annealingschritt") binden die Sonden sowohl an das erregerspezifische Amplifikat als auch an das Amplifikat der internen Kontrolle, obwohl vier Fehlpaarungen vorliegen. Danach erfolgt eine langsame Temperaturerhöhung bei kontinuierlicher Messung der Fluoreszenz. Beim Abschmelzen der Detektionssonde kommt es zu einem raschen Abfall des Fluoreszenzsignals. Figur 2 zeigt das Ergebnis der Schmelzanalyse. Aufgrund der Fehlpaarungen zeigt die Bindung der Detektionssonde an die interne Kontrolle eine wesentlich niedrigere Schmelztemperatur als die Bindung an ein Hepatitis B - PCR-Amplifikat. Die Schmelztemperatur mit interner Kontrolle betrug ca. 51°C, die Schmelztemperatur mit Hepatitis B - PCR-Amplifikat betrug etwa 73°C.
Die Figur zeigt ein Diagramm mit der ersten Ableitung der Farbstoffdetektion. Die Kurve für HBV alleine (Linie 1) steigt von 45°C ausgehend langsam an und zeigt ausschließlich einen Peak bei ca. 73°C. Die Kurve für den HBV-Test zusammen mit der internen Kontrolle (Linie 2) zeigt zwei Peaks, von denen der eine bei ca. 51°C vom Abschmelzen der Sonde von der Kontroll-DNA stammt, während der andere das Resultat der Abschmelzung der Sonde von der Erreger-DNA ist. Die Negativkontrolle mit interner Kontrolle (Linie 4) zeigt erwartungsgemäß nur einen einzigen Peak bei ca. 51°C (gestrichelte Kurve), und eine Negativkontrolle allein (Linie 3) bleibt ohne Fluoreszenz.

SEQUENCE LISTING

<110>  Labor Becker, Olgemöller & Kollegen GbR, Thorsten Becker,
       Bernd Olgemöller, Stefan Gambihler, Werner Engelhardt

<120>  Detektionsverfahren für Nucleinsäuren mit interner
       Amplifikationskontrolle

<130>  11333e

<160>  2

<170>  PatentIn version 3.2

<210>  1
<211>  188
<212>  DNA
<213>  Hepatitis B virus


<220>
<221>  source
<222>  (1)..(188)
<223>  GenX

<400>  1
gacgtccttt gtttacgtcc cgtcggcgct gaatcctgcc gacgaccctt ctcggggccg      60

cttggggctc tacgccctct ttgccgtctg ccgttccagc caaccacggg gcgcacctct     120

ctttacgcgg tctccccgtc tgtgccttct catctgccgg tccgtgtgca cttcgcttca     180

cctctgca                                                              188


<210>  2
<211>  120
<212>  DNA
<213>  Artificial sequence

<220>
<223>  This sequence is an artificial one which only partially coincides
       with GenX of Hepatitis B (SEQ ID NO:1)


<220>
<221>  source
<222>  (1)..(120)
<223>  This sequence is an artificial one which only partially coincides
       with GenX of Hepatitis B (SEQ ID NO:1)

<400>  2
gacgtccttt gtttacgtcc cgtcggcgct gaatcctgcc gacacggggc gcacctctct      60

ttacgcggtc taccagtcta tgccttatca tctgcctgtg cacttcgctt cacctctgca     120

Organization Applicant
----------------------
      Street : Führichstr. 70
      City : München
      State :
      Country : Germany
      PostalCode : 81671
      PhoneNumber :
      FaxNumber :
      EmailAddress :
<110> OrganizationName : Labor Becker, Olgemöller & Kollegen GbR, Thorsten
Becker, Bernd Olgemöller, Stefan Gambihler, Werner Engelhardt

Application Project
-------------------
<120> Title : Detektionsverfahren für Nucleinsäuren mit interner
       Amplifikationskontrolle
<130> AppFileReference : 11333e
<140> CurrentAppNumber :
<141> CurrentFilingDate : ____-__-__

Sequence
--------
<213> OrganismName : Hepatitis B virus
<400> PreSequenceString :
gacgtccttt gtttacgtcc cgtcggcgct gaatcctgcc gacgaccctt ctcggggccg      60
cttggggctc tacgccctct ttgccgtctg ccgttccagc caaccacggg gcgcacctct     120
ctttacgcgg tctccccgtc tgtgccttct catctgccgg tccgtgtgca cttcgcttca     180
cctctgca                                                              188
<212> Type : DNA
<211> Length : 188
      SequenceName : SEQ ID NO:1
      SequenceDescription :

Feature
-------
Sequence: SEQ ID NO:1:
<221> FeatureKey : source
<222> LocationFrom : 1
<222> LocationTo : 188
      Other Information : GenX
      CDSJoin : No

Sequence
--------
<213> OrganismName : Artificial sequence
<400> PreSequenceString :
gacgtccttt gtttacgtcc cgtcggcgct gaatcctgcc gacacggggc gcacctctct      60
ttacgcggtc taccagtcta tgccttatca tctgcctgtg cacttcgctt cacctctgca     120

<212> Type : DNA
<211> Length : 120
      SequenceName : SEQ ID NO:2
      SequenceDescription :

Feature
-------
Sequence: SEQ ID NO:2:
<221> FeatureKey : source
<222> LocationFrom : 1
<222> LocationTo : 120
      Other Information : This sequence is an artificial one which only
partially coincides with GenX of Hepatitis B (SEQ ID NO:1)
      CDSJoin : No

## Patentansprüche

1. Verfahren zum qualitativen oder quantitativen Nachweis einer Nucleinsäure in einer Probe mittels Amplifikation dieser Nucleinsäure und unter Verwendung einer oder mehrerer Detektions-Sonde(n), die reversibel an die amplifizierbare/ amplifizierte Nucleinsäure binden kann/können und aufgrund dieser Eigenschaft eine Detektion der nachzuweisenden Nucleinsäure ermöglichen, **dadurch gekennzeichnet, dass** das Verfahren in Gegenwart einer ebenfalls zu amplifizierenden Kontroll-Nucleinsäure in dieser Probe durchgeführt wird, die dieselbe(n) Detektions-Sonde(n) wie die nachzuweisende Nucleinsäure bindet, aber im Bindungsbereich der Sonde(n) im Vergleich zur nachzuweisenden Nucleinsäure mindestens eine Abweichung in der Nucleotid-Sequenz aufweist, derart, dass die Produkte aus nachzuweisender Nucleinsäure und Sonde(n) einerseits und Kontroll-Nucleinsäure und Sonde(n) andererseits einen unterschiedlichen Schmelzpunkt aufweisen, wobei die Temperaturdifferenz der Schmelzpunkte ausreichend groß ist, um die beiden Produkte analytisch voneinander unterscheiden zu können.

2. Verfahren nach Anspruch 1, **dadurch gekennzeichnet, dass** der Schmelzpunkt des Produktes aus Kontroll-Nucleinsäure und Sonde(n) niedriger ist als der des Produktes aus nachzuweisender Nucleinsäure und Sonde(n).

3. Verfahren nach Anspruch 1 oder 2, **dadurch gekennzeichnet, dass** die Temperaturdifferenz der Schmelzpunkte mindestens 5°C, vorzugsweise mindestens 10°C und stärker bevorzugt mindestens 15°C beträgt.

4. Verfahren nach einem der Ansprüch2 1 bis 3, **dadurch gekennzeichnet, dass** die Kontroll-Nucleinsäure mit denselben Primern amplifiziert wird wie die nachzuweisende Nucleinsäure.

5. Verfahren nach einem der voranstehenden Ansprüche, **dadurch gekennzeichnet, dass** die Amplifizierung der Nucleinsäure mit Hilfe von PCR erfolgt.

6. Verfahren nach einem der voranstehenden Ansprüche, **dadurch gekennzeichnet, dass** zwei oder mehrere Nucleinsäuren in ein und derselben Probe nachgewiesen werden, wobei das Verfahren in Gegenwart einer Kontroll-Nucleinsäure für jede nachzuweisende Nucleinsäure durchgeführt wird

7. Verfahren nach einem der voranstehenden Ansprüche, **dadurch gekennzeichnet, dass** die nachzuweisende Nucleinsäure eine DNA oder eine RNA ist, die insbesondere aus einem Krankheitserreger stammt.

8. Verfahren nach einem der voranstehenden Ansprüche, **dadurch gekennzeichnet, dass** das Vorhandensein der Nucleinsäure in der Probe mit Hilfe von "real time" Verfahren beobachtet wird.

9. Verfahren nach Anspruch 8, **dadurch gekennzeichnet, dass** die Detektion der Nucleinsäure bei einer Temperatur erfolgt, die 2-10°C unterhalb der Schmelz-Temperatur des Produktes aus nachzuweisender Nucleinsäure und Sonde(n) liegt.

10. Verfahren nach Anspruch 9, **dadurch gekennzeichnet, dass** das Produkt aus Kontroll-Nucleinsäure und Sonde(n) einen so niedrigen Schmelzpunkt besitzt, dass es bei der Detektion der nachzuweisenden Nucleinsäure im wesentlichen nicht oder gar nicht vorhanden ist.

11. Verfahren nach einem der voranstehenden Ansprüche, **dadurch gekennzeichnet, dass** nur eine Sonde verwendet wird und der Nachweis der Nucleinsäure anhand der Schmelzkurve dieser Nucleinsäure in Gegenwart der Sonde erfolgt, wobei die Schmelzkurve der Kontroll-Nucleinsäure in Gegenwart dieser Sonde als interne Kontrolle für den korrekten Ablauf der Amplifizierung dient.

12. Verfahren nach einem der Ansprüche 1 bis 10, **dadurch gekennzeichnet, dass** zwei Detektionssonden zum Nachweis der Nucleinsäure eingesetzt werden, von denen eine eine Reportergruppe trägt und die andere eine die beobachtbaren Eigenschaften der Reportergruppe verändert, wenn sie in die Nähe der Reportergruppe gelangt.

13. Verfahren nach Anspruch 12, **dadurch gekennzeichnet, dass** eine Detektionssonde zum Nachweis der Nucleinsäure eingesetzt wird, die eine Reportergruppe und eine zweite Gruppe trägt, die die beobachtbaren Eigenschaften der Reportergruppe verändert, wenn sie in die Nähe der Reportergruppe gelangt, wobei die Reportergruppe und die zweite Gruppe nur entweder während der Bindung der Detektionssonde an die Nucleinsäure oder nur im ungebundenen Zustand der Detektionssonde so nahe beieinander liegen, dass die beobachtbaren Eigenschaften der Reportergruppe verändert werden.

14. Verfahren nach einem der voranstehenden Ansprüche, **dadurch gekennzeichnet, dass** die Nucleotidsequenz der Kontroll-Nucleinsäure im Bindungsbereich der Detektions-Sonde(n) mindestens 2 Veränderungen gegenüber der nachzuweisenden Nucleinsäure aufweist, von denen vorzugsweise mindestens eine ein Austausch eines G oder eines C ist.

15. Verfahren nach einem der voranstehenden Ansprüche, **dadurch gekennzeichnet, dass** der Sequenzbereich der Kontroll-Nucleinsäure, der weder mit einer Detektions-Sonde noch ggf. mit einem Primer hybridisieren kann, verkürzt ist.

16. Verfahren nach einem der voranstehenden Ansprüche, **dadurch gekennzeichnet, dass** die Sequenz der Kontroll-Nucleinsäure in demjenigen Bereich, der weder mit einer Detektions-Sonde noch ggf. mit einem Primer hybridisieren kann, wesentliche Abweichungen gegenüber der nachzuweisenden Nucleinsäure besitzt.

17. Verwendung einer Kontroll-Nucleinsäure in einem der Verfahren gemäß gemäß Anspruch 1 bis 16, **dadurch gekennzeichnet, dass** ihre Nucleotidsequenz im Bindungsbereich der Detektions-Sonde(n) mindestens eine Veränderung gegenüber der nachzuweisenden Nucleinsäure aufweist.

18. Verwendung einer Kontroll-Nucleinsäure in einem der Verfahren gemäß gemäß Anspruch 1 bis 16, **dadurch gekennzeichnet, dass** ihre Nucleotidsequenz im Bindungsbereich der Detektions-Sonde(n) mindestens zwei, vorzugsweise drei bis fünf Veränderungen gegenüber der nachzuweisenden Nucleinsäure aufweist.

19. Verwendung einer Kontroll-Nucleinsäure in einem der Verfahren gemäß Anspruch 1 bis 16, **dadurch gekennzeichnet, dass** ihr Sequenzbereich, der weder mit einer Detektions-Sonde noch ggf. mit einem Primer hybridisieren kann, verkürzt ist.

20. Verwendung einer Kontroll-Nucleinsäure in einem der Verfahren gemäß Anspruch 1 bis 16, **dadurch gekennzeichnet, dass** ihre Sequenz in demjenigen Bereich, der weder mit einer Detektions-Sonde noch ggf. mit einem Primer hybridisieren kann, wesentliche Abweichungen gegenüber der nachzuweisenden Nucleinsäure besitzt.

21. Verwendung einer Kontroll-Nucleinsäure nach Anspruch 18, **dadurch gekennzeichnet, dass** die Veränderungen ungefähr gleichmäßig über den Bindungsbereich der Detektions-Sonde(n) verteilt sind. ,

22. Kit, umfassend eine Nucleinsäure, die insbesondere als Kontroll-Nucleinsäure für die Negativkontrolle in einem Verfahren zum Nachweisen einer gesuchten Nucleinsäure geeignet ist, sowie ein Sondensystem mit einer oder mehreren oligonucleotidhaltigen Sonden, dessen Sonde(n) an die Kontroll-Nucleinsäure binden kann/können und das eine Reportergruppe mit einer beobachtbaren Eigenschaft aufweist, die sich in Abhängigkeit davon ändert, ob die Sonde(n) des Systems an dieser Nucleinsäure gebunden ist/sind oder nicht, **dadurch gekennzeichnet, dass** die insbesondere als Kontroll-Nucleinsäure geeignete Nucleinsäure in mindestens einem derjenigen Bereiche, in denen das/die Oligonucleotide der Sonde(n) des Sondensystems binden können, mindestens eine Fehlbindungsstelle gegenüber dem in diesem Bereich bindenden Oligonucleotid der Sonde(n) aufweist.

23. Kit nach Anspruch 22, worin die insbesondere als Kontroll-Nucleinsäure geeignete Nucleinsäure mindestens 2, bevorzugt drei bis 5 Fehlbindungsstellen aufweist.

24. Kit nach einem der Ansprüche 22 oder 23, worin die Fehlstellen der insbesondere als Kontroll-Nucleinsäure geeigneten Nucleinsäure im Wesentlichen gleichmäßig über den Bindungsbereich des Oligonucleotids der Sonde(n) verteilt angeordnet sind.
